# EUROPÄISCHE PATENTANMELDUNG

(11) **EP 0 836 095 A2**
(43) Veröffentlichungstag der Anmeldung: **15.04.1998**
(21) Anmeldenummer: 97117472.7
(22) Anmeldetag: 09.10.1997
(51) Int. Cl.: G01N 33/42, G01N 5/04

(54) **Vorrichtung zur Ermittlung eines thermisch zersetzbaren Anteils einer Probe**

(30) Priorität: 11.10.1996 DE 19641983
(71) Anmelder: Isa-Techniek Anlagenbau GmbH, 63755 Alzenau (DE)
(72) Erfinder: Laubenthal, Franz, 63486 Bruchköbel (DE); Salber, Jozef, 63755 Alzenau (DE)
(74) Vertreter: Stoffregen, Hans-Herbert, Dr. Dipl.-Phys.

(57) **Zusammenfassung**

Die Erfindung bezieht sich auf eine Vorrichtung zur Ermittlung eines thermisch zersetzbaren Anteils wie Bindemittel einer Probe, insbesondere eines bituminösen Baustoffs wie Asphaltprobe, umfassend einen Probenraums (24) mit Probenraumseiten-, -boden-, -decken- und - rückwandung (26, 28, 30, 32, 34) sowie vorzugsweise über eine Tür (22) verschließbarer Öffnung und einer Probenaufnahme (58), wobei der Probenaufnahme eine Heizung (78, 80, 82, 100, 102, 104) zugeordnet ist, insbesondere eine flächig abstrahlende ober- und/oder unterhalb der Probenaufnahme angeordnete Heizung. Um die Probe auf kleinstem Raum großflächig verteilen zu können, wird vorgeschlagen, daß die Probenaufnahme (58) als in dem Probenraum (24) drehbar angeordnete Trommel ausgebildet ist.

## Beschreibung

Die Erfindung bezieht sich auf eine Vorrichtung zur Ermittlung eines thermisch zersetzbaren Anteils wie Bindemittel einer Probe, insbesondere eines bituminösen Baustoffs wie Asphaltprobe, umfassend einen Probenraum mit Probenraumseiten-, boden-, decken- und rückwandungen sowie vorzugsweise über eine Tür verschließbaren Öffnung und einer insbesondere mit einer Wiegeeinrichtung verbindbaren Probenaufnahme, wobei der Probenaufnahme zumindest eine Heizung zugeordnet ist, insbesondere eine flächig abstrahlende ober- und/oder unterhalb der Probenaufnahme angeordnete Heizung.

Aus der DE 38 08 888 A 1 oder der DD 113 407 ist ein Verfahren sowie eine Vorrichtung zur Ermittlung des Bindegehalts von bituminösen Baustoffen bekannt, wobei der Bindemittelgehalt durch thermische Zersetzung bestimmt wird. Hierzu wird eine Probe gewogen, sodann das Bindemittel aus der Probe entfernt, die Probe erneut gewogen und schließlich der Bindemittelgehalt aus der Gewichtsdifferenz bestimmt. Nach der EP 0 333 063 A 2 wird die Probe dabei auf eine Probenaufnahme gelegt, die über ein Gestänge mit einer unterhalb des Ofens vorhandenen Waage zur fortlaufenden Gewichtsmessung verbunden ist, um den zeitlichen Verlauf der Gewichtsänderung zu bestimmen.

Um innerhalb kurzer Zeitspannen verschiedene Proben prüfen zu können, wird nach der WO 94/23279 ein Kaltwandofen benutzt. Das Probengut selbst wird - wie bei den anderen bekannten Vorrichtungen - flächig auf der Probenaufnahme ausgebreitet. Dabei kann die Probenaufnahme selbst mit einer Rüttelvorrichtung verbunden sein.

Um ein räumlich gleichmäßiges Temperaturfeld im Probengut bei im wesentlichen ausschließlicher Erwärmung des Probengutes selbst zu gewährleisten, ist nach dem DE 94 19 440 U 1 vorgesehen, als Heizung flächig abstrahlende Infrarot-Heizelemente zu verwenden, die sowohl unter- als auch oberhalb der Probengutaufnahme angeordnet sind.

Aus der US 4,439,209 ist eine Vorrichtung zur thermischen Zersetzung organischer Produkte bekannt, die eine zylindrische Drehtrommel mit achsial in dieser verlaufenden Welle umfaßt.

Der vorliegenden Erfindung liegt die Aufgabe zugrunde, mit konstruktiv einfachen Maßnahmen den thermisch zersetzbaren Anteil einer Probe, insbesondere den Bindemittelgehalt einer Asphaltprobe zu bestimmen, wobei das Probengut auf engstem Raum großflächig verteilt werden soll. Dabei soll sichergestellt sein, dass die Probengutbestandteile möglichst gleichmäßig der Heizung ausgesetzt werden. Auch soll eine Vereinfachung des Wägeprozesses sowie eine problemlose Klassierung der Probenbestandteile möglich sein.

Das Problem wird erfindungsermäß im wesentlichen dadurch gelöst, dass die Probenaufnahme als in dem Probenraum drehbar angeordnete Trommel ausgebildet ist, wobei insbesondere die Trommel umfangsseitig und in einer (ersten) Stirnwand geschlossen ist.

Abweichend vom vorbekannten Stand der Technik wird das Probengut nicht auf einer Probenaufnahme flächig ausgebreitet, sondern in eine Trommel eingebracht, die ihrerseits vorzugsweise um ihre horizontal verlaufende Längsachse drehbar ist. Hierdurch erfolgt eine scheinbare Vergrößerung der von der Heizung bestrahlten Probenoberfläche, so dass in relativ kurzer Zeit eine thermoanalytische Bestimmung der zersetzbaren Bestandteile des zu prüfenden Gutes erfolgen kann.

Zum Drehen der Trommel kann diese auf Gleitelementen wie Rollen ruhen, wobei zumindest ein Gleitelement zwangsangetrieben ist. Folglich ist mit konstruktiv einfachen Maßnahmen eine Drehung der Trommel möglich, wobei die Trommel selbst auf die entsprechenden Gleit- bzw. Antriebselemente wie -rollen aufgesetzt wird, so dass ein problemloses Einbringen der Trommel mit dem zu überprüfenden Gut und ein Entfernen der Trommel nach dem thermischen Zersetzungsprozeß erfolgen kann, ohne dass eine Kupplung oder ähnliches erforderlich ist.

Um das Probengut im erforderlichen Umfang auf der Innenfläche der Trommel auszubreiten und im gewissem Umfang mit der Trommelbewegung mitzunehmen, sieht eine Weiterbildung der Erfindung vor, dass die Trommelumfangswandung zumindest bereichsweise strukturiert ist. Dies kann durch Ausbildung von Vorsprüngen bzw. Vertiefung der geschlossenen Trommelumfangswandung erfolgen.

Eine konstruktive Vereinfachung ergibt sich des weiteren daraus, dass die der geschlossenen ersten Stirnwand gegegenüberliegende zweite Stirnwand offen ist', die gegebenenfalls von einer stationär angeordneten Heizung durchsetzbar ist, die vorzugsweise von der Rückwandung des Probenraums ausgeht. Bei der Heizung kann es sich um Infrarotflächenheizelemente handeln, die probenabgewandt von einer Abdeckung abgeschirmt ist, um gegebenenfalls herabfallende Probenteile von der Heizung fernzuhalten, wodurch andernfalls eine Verfälschung der Meßergebnisse erfolgen würde.

Nach einer alternativen Ausführungsform können Heizelemente, insbesondere flächige Heizelemente außerhalb der Trommel winkelig zur Horizontalen angeordnet sein. Dabei sollte die von den in einer Reihe angeordneten und eine Ebene aufspannenden Heizelementen symmetrisch zur vertikal verlaufenden Mittelebene der Trommel verlaufen und vorzugsweise einen Winkel von in etwa 90° einschließen. Auch können oberhalb der Trommel Heizelemente angeordnet sein.

In den von der vorzugsweise dachförmig ausgebildeten Abdeckung umgebenden Raum kann -sofern erforderlich- von der Rückseite des Probenraums her Gas wie N₂ oder Inertgas, gegebenenfalls auch Luft in das Trommelinnere eingeführt werden, wobei das Gas über seitliche Öffnungen der Abdeckung in die Trommel strömt, um sodann über die Öffnung der zweiten Stirnwandung in den Probenraum zu gelangen.

Losgelöst hiervon wird Rauchgas über in der Deckenwandung des Probenraums vorhandene Öffnung bzw. Öffnungen mittels eines außerhalb des Probenraums angeordneten Gebläses abgesaugt. Um den Durchsatz zu regulieren, wodurch auch eine Temperatureinstellung im Probenrauminneren erfolgt, ist dem Gebläse eine Leitung bzw. ein Einsatz vorgeschaltet, der z.B. über eine Blende verschließbare Öffnung zum Ansaugen von Falschluft aufweist.

Um die Trommel anzutreiben, sieht eine Weiterbildung der Erfindung vor, dass ein Kettenrad mittels eines außerhalb des Probenraums angeordneten Drehantriebs antreibbar ist, das mit einer die Trommel umfangsseitig umgebenden Kette wechselwirkt. Selbstverständlich kann in die Trommel umfangsseitig auch eine Verzahnung eingeprägt sein, die mit dem Kettenrad wechselwirkt. Als weitere Alternative ist ein Antrieb über ein Reibrad denkbar.

Das Antriebsrad selbst geht von einer Welle aus, auf der vorzugsweise eine weitere Abstützrolle für die Trommel gelagert ist. Von einer weiteren Welle gehen ebenfalls Abstützrollen aus, auf die die Trommel aufsetzbar ist, um gedreht werden zu können.

Die Wellen gehen von einem in den Probenraum einbringbaren Gestell oder Rack aus, von dem gegebenenfalls zusätzlich eine Flächenheizung wie IR-Flächenstrahler ausgeht, die unterhalb der Trommel verläuft. Somit kann das Probengut sowohl von oben als auch von unten beheizt werden.

Unterhalb des Probenraums ist die Wiegeeinrichtung angeordnet, die eine Waageplatte aufweist, von der Stempel oder Bolzen ausgehen, die den Probenboden durchsetzen und zur Trommel führen. Hierzu sind Öffnungen bzw. Durchführungen im Boden vorhanden, die gleichzeitig der Zuluftzuführung dienen. Weitere Zuluftöffnungen sind gegebenenfalls nicht erforderlich. Die Wiegeeinrichtung ist anheb- und absenkbar. Dies erfolgt vorzugsweise mittels eines Exzenters, der mit der Wiegeeinrichtung zusammenwirkt und mittels eines Hubantriebs betätigbar ist. Wird die Wiegeeinrichtung und damit die Waageplatte angehoben, so erfassen die Stempel oder Bolzen die Trommel umfangsseitig und heben diese von den Antriebs- und Gleitelementen wie -rollen ab, so dass die Trommel einschließlich des sich in dieser befindlichen Probengutes gewogen werden kann. Wird die Wiegeeinrichtung mittels des Hubantriebs abgesenkt, so liegt die Trommel ausschließlich auf den Antriebs- und Gleitelementen auf, so dass die erforderliche Drehung durchgeführt werden kann.

Um ein geführtes Anheben bzw. Absenken der Wiegeeinrichtung sicherzustellen, weist diese Führungshülsen auf, die ihrerseits Führungsbolzen umgeben, die vom Boden des Gehäuses ausgehen, innerhalb der sich der Probenraum befindet.

Das Gehäuse selbst ist vorzugsweise als Schrank ausgebildet und weist einen aus Edelstahl bestehenden Innen- und Außenmantel auf, die gegeneinander vorzugsweise über Mineralfasern isoliert sind und den Probenraum umgeben.

In dem die Wiegeeinrichtung aufnehmenden Raum unterhalb des Probenraums ist des weiteren der Drehantrieb für das Antriebsrad angeordnet.

Andere Antriebsarten sind gleichfalls möglich wie z.B. eine Magnetkupplung. Insoweit wird jedoch auf hinlänglich bekannte Konstruktionen und Lösungsmöglichkeiten verwiesen.

Um nach dem thermischen Zersetzungsprozeß ein Klassieren des verbliebenen Probenguts vorzunehmen, sieht ein weiterer vorzuhebender Vorschlag der Erfindung vor, dass die die Öffnung aufweisende Stirnwandung einen umlaufenden Kragen aufweist, auf den ein Sieb bzw. ein Siebsatz aufsetzbar ist. Dieses Sieb bzw. der Siebsatz wird auf den Kragen aufgesetzt, nachdem die Trommel aus dem Probenraum entfernt und zunächst auf der geschlossenen Stirnwand abgestellt ist. Wird sodann die Trommel um 180 ° gedreht, so kann das Probengut das Sieb bzw. den Siebsatz durchsetzen, um die erforderliche Klassierung zu erreichen.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines der Zeichnung zu entnehmenden bevorzugten Ausführungsbeispiels.

Es zeigen:
- Fig. 1: einen Querschnitt durch ein Thermoanalysegerät,
- Fig. 2: einen Schnitt entlang der Linie II - II in Fig. 1,
- Fig. 3: einen Verfahrensablauf zum Klassieren von Probengut nach erfolgter thermischer Zersetzung,
- Fig. 4: einen Querschnitt durch eine alternative Auführungsform eines Thermoanalysegerätes nach der Fig. 1 und
- Fig. 5: eine Variante des Thermoanalysegerates gemäß Fig. 4.

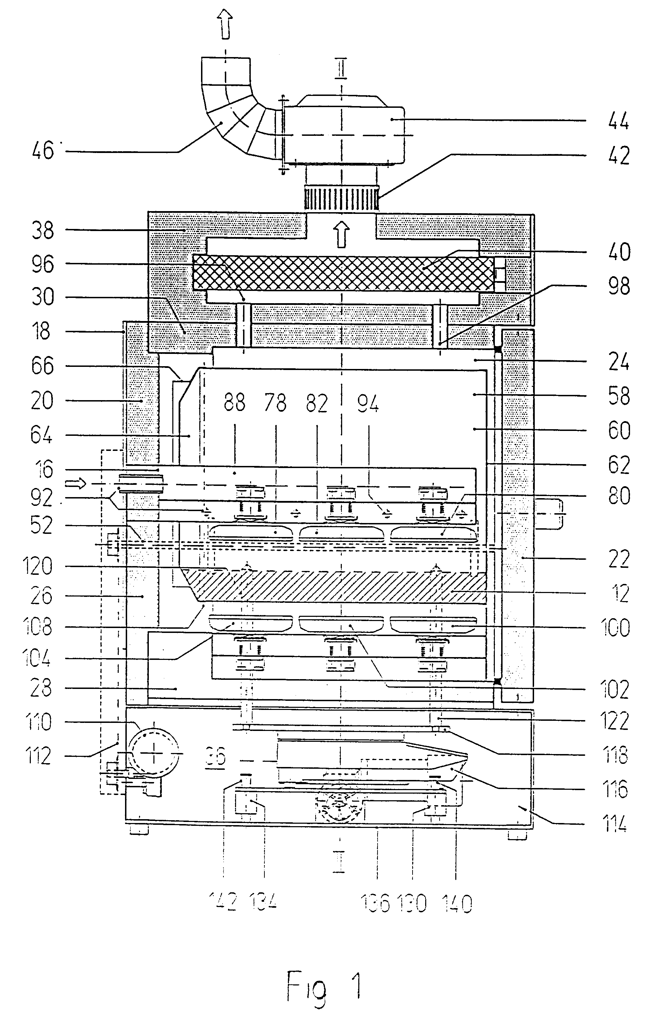

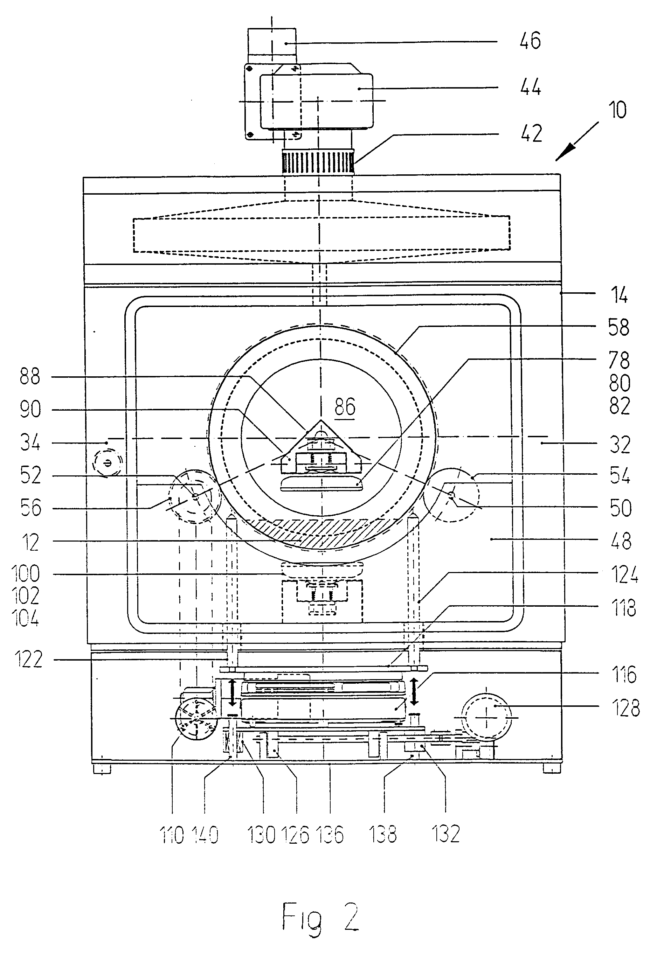

Die Fig. 1 und 2 zeigen eine Prinzipdarstellung eines Thermoanalysegerätes 10, mit Hilfe dessen ein thermisch zersetzbarer Anteil einer Probe, insbesondere der Bindemittelgehalt einer Asphaltprobe 12 bestimmt werden soll.

Das Thermoanalysegerät 10 umfaßt ein schrankförmiges Gehäuse 14 mit Innen- und Außenwandung 16 und 18, die aus Edelstahl bestehen und gegeneinander vorzugsweise mittels Mineralfasern 20 isoliert sind. Das Gehäuse 14 ist über eine Tür 22 verschließbar, über die ein Probenraum 24 zugänglich ist, der neben der Tür 22 von einer Rückwandung 26, einer Bodenwandung 28, einer Deckenwandung 30 sowie Seitenwandungen 32 und 34 begrenzt wird.

Unterhalb des Probenraums 24 und der Bodenwandung 28 ist in dem schrankförmigen Gehäuse 14 ein Gehäuseunterteil 36 mit Wäge- und Steuerteil sowie Antrieben vorgesehen, auf die nachstehend näher eingegangen wird.

Das Gehäuse 14 weist ferner einen Aufsatz 38 mit einem Rußfilter 40 auf. Von dem Aufsatz 38 geht ein Stutzen 42 aus, der zu einem Gebläse 44 führt, über das Abgas über einen Auslaß 46 abströmt.

In dem Probenraum 24 ist ein als Rack bezeichnetes Gestell 48 angeordnet, das zwei parallel zueinander und zu der Frontseite des Gehäuses 14 verlaufende Schenkel aufweist, zwischen denen Wellen 50, 52 verlaufen, die Stützrollen 54 sowie ein Antriebsrad 56 tragen. Vorzugsweise weist die Welle 50 zwei Stützrollen und die Welle 52 neben dem Antriebsrad 56 eine weitere Stützrolle auf. Selbstverständlich können auf jeder Welle 50, 52 auch eine von zwei abweichende Anzahl von Rollen gelagert sein.

Auf die Stütz- und Antriebsrollen 54, 56 ist eine Probenaufnahmetrommel 58 aufsetzbar, die eine geschlossene Umfangswandung 60 und eine geschlossene erste Stirnwandung 62 aufweist, die bei in dem Probenraum 24 eingebrachter Trommel 58 zur Tür 22 weist. Die Mittelachse der Trommel 58, die mit dessen Drehachse zusammenfällt, verläuft horizontal.

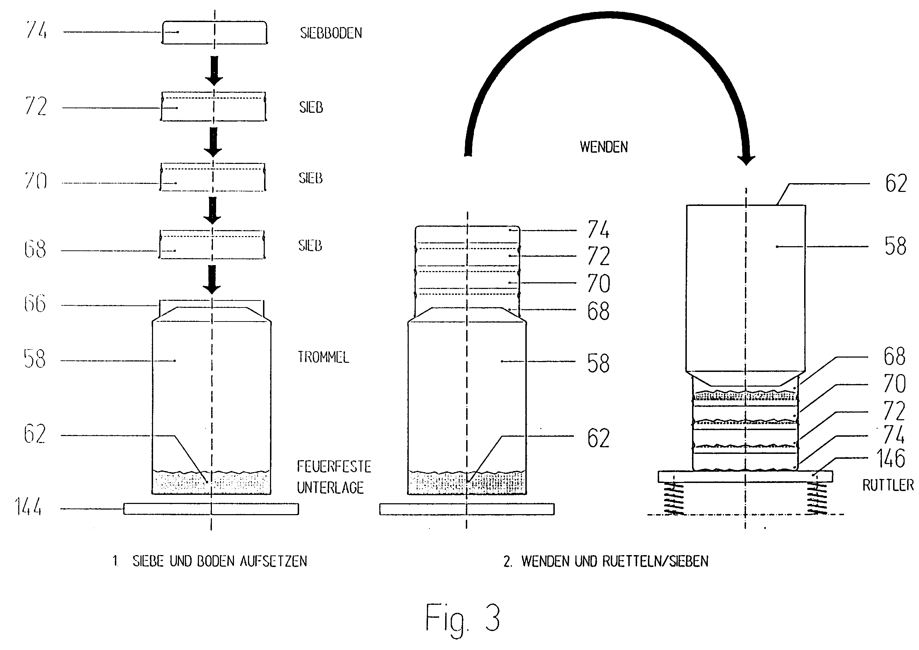

Rückseitig ist die Trommel 58 offen, weist also eine Öffnung 64 auf, die von einem umlaufenden Kragen 66 umgeben ist, auf den entsprechend der Fig. 3 ein aus mehreren Sieben 68, 70, 72 aufsetzbarer Siebsatz mit Siebboden 74 aufsetzbar ist, um eine Klassierung des Probenguts 12 nach erfolgter thermischer Zersetzung vornehmen zu können.

Parallel zur Drehachse und vorzugsweise mit dieser zusammenfallend erstreckt sich von der Rückwandung 20 ausgehend eine Halterung 76, von der die IR-Flächenstrahler, im Ausführungsbeispiel drei Flächenstrahler 78, 80, 82 ausgehen. Die Halterung 76 durchsetzt die Öffnung 64 der Trommel 58, sofern diese auf den Stütz- und Antriebsrollen 54, 56 aufgesetzt ist.

Die Infrarotstrahler 78, 80, 82 sind von einer dachförmigen Abdeckung 88 probenabgewandt abgeschirmt, um gegebenenfalls beim Drehen der Trommel 58 mitgerissenes Probengut von den Infrarotstrahlern 78, 80, 82 fernzuhalten.

In dem von der Abdeckung 88 umgebenden Raum 90 kann gegebenenfalls eine die Gehäuserückwandung 20 durchsetzende Öffnung 92 münden, über die insbesondere N₂ oder Inertgas, gegebenenfalls auch Zuluft zuführbar ist. Das Gas gelangt sodann von dem Raum 90 über seitliche Öffnungen 92, 94 der Abdeckung 88 in den Trommelinnenraum 86, um sodann -wie Rauchgas- über die Öffnung 64 in den Probenraum 24 und über in der Deckenwandung 30 vorhandene Öffnungen 96, 98 zu strömen. Die Abluft wird dabei mittels des Gebläses 46 duch den Rußfilter 40 gesaugt, um zur Abgasleitung 46 zu gelangen. Die Menge der durch den Probenraum 24 gesaugten Zuluft kann durch eine den Stutzen 42 umgebende, jedoch nicht dargestellte Lochblende dadurch eingestellt werden, dass in Abhängigkeit von den im Querschnitt veränderbaren Öffnungen in dem Stutzen 42 gewünschte Mengen von Falschluft mit abgesaugt werden.

Von dem Gestell 48 gehen des weiteren unterhalb der Trommel 58 verlaufende Heizungen 100, 102 und 104 aus, bei denen es sich ebenfalls um Infrarotflächenheizelemente handeln kann.

Das Antriebsrad 56 ist im Ausführungsbeispiel als Kettenrad ausgebildet, das mit einer die Trommel 58 umfangsseitig umgebenden Kette 106 kämmt. Eine andere Antriebsmöglichkeit z.B. über ein Reibrad ist gleichfalls möglich. Auch kann die Trommel 58 peripher Vertiefungen und Erhebungen aufweisen, die mit dem Kettenrad 56 wechselwirken.

Unabhängig davon sollte die geschlossene Trommelumfangswand 60 durch z.B. Vertiefungen und Erhebungen strukturiert sein, um beim Drehen Probengut mitzunehmen, das schwerkraftsbedingt sodann zurückfällt.

Um das Kettenrad 56 zu drehen, wird die Welle 52 mittels eines Drehantriebs 110 in Drehbewegung gesetzt. Das entsprechende Übertragungselement wie Riemen oder Kette 112 verläuft im Ausführungsbeispiel außerhalb des Gehäuses 14, ohne dass dies jedoch zwingend erforderlich ist.

Der Drehantrieb 110 ist in dem Gehäuseuntersatz 36 genauso wie eine Wiegeeinrichtung 114 angeordnet. Die Wiegeeinrichtung 114 umfaßt eine Waage 116 mit Waageplatte 118, von der Stempel oder Bolzen 120, 122, 124 ausgehen, die in Abhängigkeit von der Position der Waage 116 die Trommel 58 von den Stütz- und Antriebsrollen 54, 56 abheben, um eine Wägung durchzuführen, oder in Ausgriff mit der Trommel 58 gelangen, so dass diese auf den Stütz- und Antriebsrollen 54, 56 ruht und gedreht werden kann. Dabei durchsetzen die Stempel oder Bolzen 120, 122, 124 beabstandet nicht näher bezeichnete Öffnungen im Boden 28 des Gehäuses 14, über die auch die erforderliche Menge an Zuluft strömen kann.

Die Waage 116 ist über einen Exzenter 126 mit einem Hubantrieb 128 verbunden, der sich ebenfalls in dem Gehäuseuntersatz 36 befindet. Um gegebenenfalls zu Verfälschungen des Wiegeprozesses führendes Anheben bzw. Absenken der Waage 116 auszuschließen, gehen von der Waage 116 Führungshülsen 130, 132, 134 aus, die die vom Boden 136 des Untersatzes 36 ausgehende Führungsbolzen 140, 142, 147 umgeben.

Soll der Bindemittelgehalt einer Asphaltprobe 12 bestimmt werden, so wird das Probengut 12 zunächst in die Trommel 58 außerhalb des Gehäuses 14 eingebracht. Anschließend wird die Trommel 58 auf die Stütz- und Antriebsrollen 54, 56 aufgesetzt und die Tür 22 geschlossen.

Nachdem die Probe 12 über einen vorgegebenen Zeitraum von z.B. 5 Minuten einer Temperatur ausgesetzt ist, die ein Verdampfen von Restfeuchtigkeit aus dem Probengut 12 sicherstellt, erfolgt eine erste Gewichtsmessung. Hierzu wird die Waage 116 mittels des Exzenters 126 angehoben, so dass über die Stempel 120, 122, 124 die Trommel 58 zu den Stütz- und Antriebsrollen 54, 56 beabstandet wird. Nach Durchführung der Messung wird die Trommel 58 durch Absenken der Waage 116 auf die Rollen 54, 56 wieder aufgesetzt und durch Betätigung des Drehantriebes gedreht. Die Umdrehungsgeschwindigkeit sollte dabei in etwa eine Umdrehung pro Minute sein. Umdrehungsgeschwindigkeiten bis zu 5 Umdrehungen pro Minuten sind gleichfalls denkbar. Nach einer vorgegebenen Zeit von z.B. 30 Minuten wird der Drehantrieb 110 ausgeschaltet, um eine weitere Messung vorzunehmen. Nach erfolger Messung kann über einen weiteren Zeitraum von z.B. 3 Minuten die Trommel 58 erneut gedreht werden, um sodann eine weitere Messung vorzunehmen. Sofern zwischen der zweiten und dritten Messung eine Gewichtsveränderung im merklichen Umfang nicht mehr feststellbar ist (Gewichtsänderung kleiner als 0,1 g) kann davon ausgegangen werden, dass der thermisch zersetzbare Anteil des Probengutes 12, also bei einer Asphaltprobe das Bitumen entfernt ist. Sodann wird die Heizung abgeschaltet und die Tür 22 geöffnet, um die Trommel 58 herauszunehmen. Die Trommel wird sodann auf eine feuerfeste Platte 144 mit der geschlossenen Seite 62 gestellt. Auf den die Öffnung 64 umgebenden Kragen 66 wird ein Siebsatz aufgesetzt, der im Ausführungsbeispiel aus den Sieben 68, 70, 72 unterschiedlicher Maschenweite und dem Siebboden 74 besteht. Sodann wird die Trommel 58 um 180 ° gedreht und mit dem Siebboden 74 auf einen Rütteltisch 146 aufgesetzt, damit das Probengut die Siebe 68, 70. 72 durchsetzen kann, wodurch eine Klassierung erfolgt.

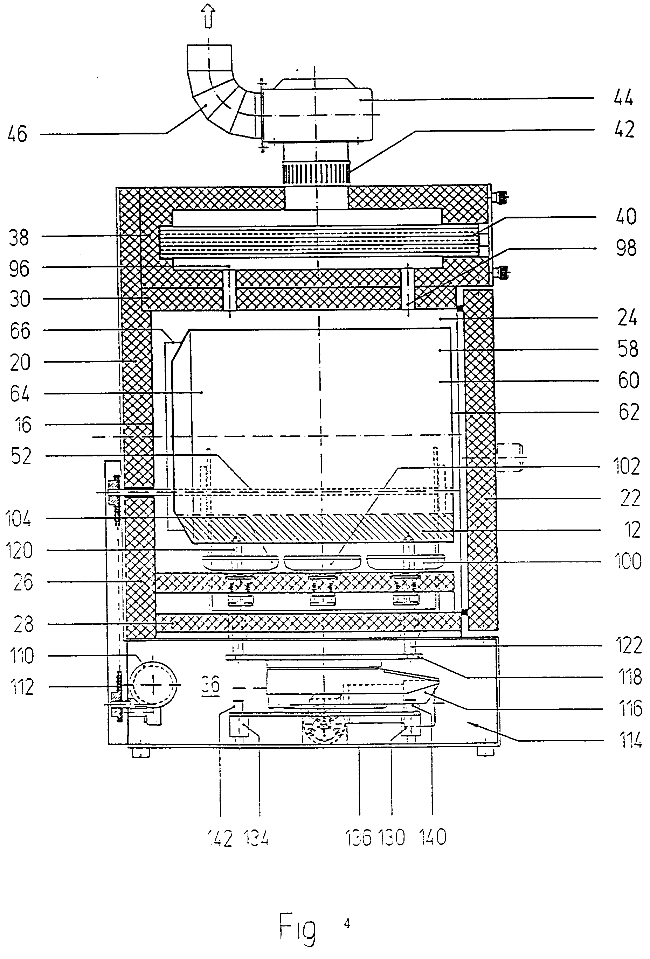

Das Ausführungsbeispiel der Fig. 4 unterscheidet sich von dem der Fig. 1 dahingehend, dass sich flächige Heizelemente 100, 102, 104 nur unterhalb der Trommel 58, und nicht innerhalb dieser erstrecken.

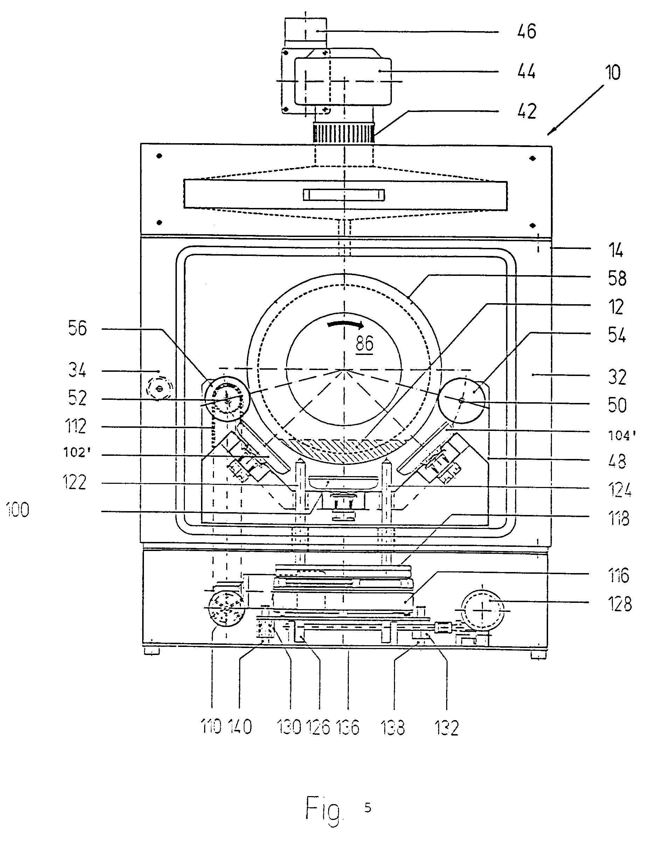

Beim Ausführungsbeispiel der Fig. 5, das eine Variante zu dem der Fig. 4 darstellt, sind unterhalb der Trommel 58 flächige Heizstrahler 100, 102 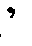 , 104 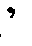 , wobei neben einer zentral unterhalb der Längsachse der Trommel 58 verlaufenden und von den Heizstrahlern 100 gebildete Heizlinien zwei symmetrisch zu dieser und von den Heizstrahlern 102 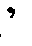 , 104 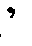 gebildete Heizlinien seitlich entlang der Trommelaußenwandung angeordnet sind. Dabei spannen die Heizstrahler 102 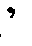 , 104 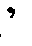 Ebenen auf, die sich unter einem Winkel von in etwa 90° schneiden.

## Patentansprüche

1. Vorrichtung zur Ermittlung eines thermisch zersetzbaren Anteils wie Bindemittel einer Probe, insbesondere eines bituminösen Baustoffs wie Asphaltprobe, umfassend einen Probenraums (24) mit Probenraumseiten-, -boden-, -decken- und -rückwandungen (26, 28, 30, 32, 34) sowie vorzugsweise über eine Tür (22) verschließbarer Öffnung und einer Probenaufnahme (58), wobei der Probenaufnahme eine Heizung (78, 80, 82, 100, 102, 104) zugeordnet ist, insbesondere eine flächig abstrahlende ober- und/oder unterhalb der Probenaufnahme angeordnete Heizung,
**dadurch gekennzeichnet**,
dass die Probenaufnahme (58) als in dem Probenraum (24) drehbar angeordnete Trommel ausgebildet ist.

2. Vorrichtung nach Anspruch 1,
**dadurch gekennzeichnet**,
dass die Trommel (58) umfangsseitig und in einer (ersten) Stirnwand (62) geschlossen ist, die vorzugsweise bei in dem Probenraum (24) angeordneter Trommel der Tür (22) zugewandt ist, und dass vorzugsweise die der ersten Stirnwand (60) gegenüberliegende (zweite) Stirnwand offen ist, die gegebenenfalls bei in dem Probenraum (24) angeordneter Trommel (58) von einer Heizung (78, 80, 82) oder einem Träger (76) für eine solche durchsetzt ist, wobei insbesondere die Heizung (78, 80, 82) bzw. deren Halterung (76) von der Probenraumrückwandung (26) ausgeht.

3. Vorrichtung nach zumindest einem der vorhergehenden Ansprüche,
**dadurch gekennzeichnet**,
dass die Trommel (58) innerhalb des Probenraums (24) auf Sützelementen wie Sützrollen (54) sowie zumindest einem Antriebselement wie Antriebsrolle (56) drehbar abgestützt ist, wobei insbesondere die Antriebsrolle (56) ein Kettenrad ist, das in eine umfangsseitig die Trommel (58) umgebende Kette (62) eingreift, oder die Antriebsrolle (56) ein Reibrad ist, und dass vorzugsweise die Antriebsrolle (56) von einer außenseitig vom Probenraum (24) angetriebenen Welle (52) ausgeht.

4. Vorrichtung nach zumindest einem der vorhergehenden Ansprüche,
**dadurch gekennzeichnet**,
dass die sich innerhalb der Trommel (58) erstreckende Heizung eine Flächenheizung, insbesondere Infrarotflächenheizung (78, 80, 82) ist, die probenabgewandt von einer vorzugsweise dachförmigen Abdeckung (88) abgeschirmt ist, wobei vorzugsweise eine Gaszuführöffnung (92) von der Gehäuserückwand (20) ausgeht, die in den von der Abdeckung (88) der Heizung (78, 80, 82) umgebenden Raum (90) führt, wobei das Gas über seitliche Öffnungen (92, 94) der Abdeckung über die offene zweite Stirnseite der Trommel (58) über zumindest eine in der Deckenwandung (30) des Probenraums (24) vorhandene Öffnung (96, 98) strömt.

5. Vorrichtung nach zumindest einem der vorhergehenden Ansprüche,
**dadurch gekennzeichnet**,
dass die in der Deckenwandung (30) vorhandene Öffnung (96, 98) vorzugsweise über einen Rußfilter (40) mit einem Gebläse (44) verbunden ist, wobei über das Gebläse sowohl Abluft (Rauchgas) des Probenraums (24) als auch Falschluft zur Regulierung der Abluftmenge saugbar ist.

6. Vorrichtung nach zumindest einem der vorhergehenden Ansprüche,
**dadurch gekennzeichnet**,
dass eine Wiegeeinrichtung (114) unterhalb des Probenraums (24) vorzugsweise in einem Gehäuseuntersatz (36) angeordnet ist, dass von der Wiegeeinrichtung Abstützungen wie Bolzen oder Stempel (120, 124, 126) zum Anheben bzw. Absenken der Probenguttrommel (58) ausgehen und Öffnungen in der Probenraumbodenwandung (28) beabstandet durchsetzen, wobei die Öffnungen vorzugsweise Zuluftöffnungen sind, dass die Wiegeeinrichtung eine anheb- bzw. absenkbare Waageeinheit (116) mit Waageplatte (118) umfaßt, von der die die Probenraumbodenwandung (28) durchsetzenden Stempel bzw. Bolzen (120, 122, 124) ausgehen.

7. Vorrichtung nach zumindest einem der vorhergehenden Ansprüche,
**dadurch gekennzeichnet**,
dass die Wiegeeinrichtung mittels eines Hubantriebs (128), insbesondere mittels eines von diesem betätigten Exzenters (126) anheb- bzw. absenkbar ist, und dass insbesondere die Wiegeeinrichtung Führungshülsen (130, 132, 134) aufweist, die vom Boden des Gehäuseunterteils (36) ausgehende Führungsbolzen (138, 140, 142) umgeben.

8. Vorrichtung nach zumindest einem der vorhergehenden Ansprüche,
**dadurch gekennzeichnet**,
dass unterhalb der Trommel (58) eine flächige Heizung insbesondere IR-Flächenheizelemente (100, 102, 104) angeordnet ist und/oder dass seitlich der Trommel und vorzugsweise symmetrisch zu deren Mittellängsebene flächige Heizelemente (102 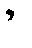 , 104 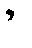 ) wie IR-Heizelement angeordnet sind.

9. Vorrichtung nach zumindest einem der vorhergehenden Ansprüche,
**dadurch gekennzeichnet**,
dass die Trommel (58) in ihrer Umfangswandung (60) zumindest bereichsweise strukturiert ist und/oder dass die Trommel in Bezug auf die offene zweite Stirnwand einen umlaufenden Kragen (66) als Aufnahme für ein Sieb bzw. einen Siebsatz (68, 70, 72, 74) aufweist.

10. Vorrichtung nach zumindest einem der vorhergehenden Ansprüche,
**dadurch gekennzeichnet**,
dass innerhalb des Probenraums (24) ein Gestell (48) angeordnet ist, von der die die Antriebs- und Abstützrollen (54, 52) lagernden Wellen (50, 52) ausgehen, wobei vorzugsweise von dem Gestell die unterhalb der Trommel (58) verlaufende Heizung (100, 102, 104) ausgeht.
